# EUROPEAN PATENT APPLICATION

(11) **EP 4 570 866 A1**
(43) Date of publication of application: **18.06.2025**
(21) Application number: 23315454.1
(22) Date of filing: 14.12.2023
(51) Int. Cl.: C09D 11/00, C07D 403/12, C07C 211/00

(54) **INK COMPOSITION FOR STAMP-MARKING ON AN OPHTHALMIC LENS**

(71) Applicant: ESSILOR INTERNATIONAL, 92440 Charenton-Le-Pont (FR)
(72) Inventor: YU, Hui, 122210 SINGAPORE (SG); BHANGALE, Sunil Madhukar, 689682 SINGAPORE (SG)
(74) Representative: LLR

(57) **Abstract**

An ink composition for stamp-marking on an ophthalmic lens, said composition comprising at least:
- a compound of formula (I) 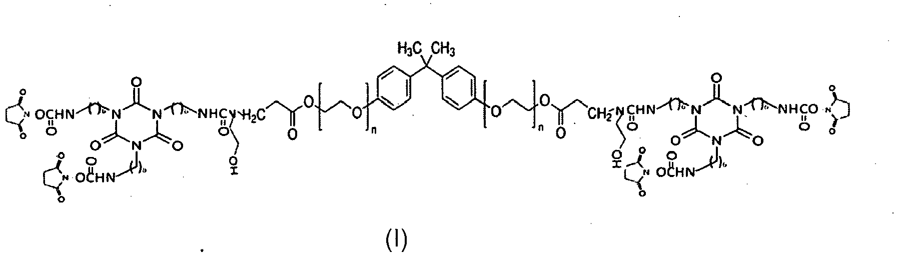 wherein n is 1 or 2,
- a drying aid, and
- at least one pigment.

## Description

### TECHNICAL FIELD

The present disclosure relates to an ink composition for stamp-marking ophthalmic lenses.

### BACKGROUND OF THE DISCLOSURE

Inks are used to mark ophthalmic lenses to provide either technical information, e.g. about the confocal or biconfocal point, or commercial information e.g.; branding and so on. The ink should have good compatibility with the stamp used and provide good adhesion on the lens. Also, it needs to be easily removable when such removal is needed, e.g. at the optical manufacturing plant or laboratory or by the eyecare practitioner (ECP). The ink, after removal, should not leave trace or ghost image on the lens.

Ghost image refers to the optical distortion created by the ink on the lens surface after its removal. It is observed under various light conditions (R17 or ARC lamp) after removal of the ink printed on the lens.

Inks used for conventional stamp pads use castor oil and derivatives thereof as principal solvents, or glycols and glycerin. Accordingly, the vapor pressures are extremely low, and the drying speeds are very slow. This drying speed makes difficult to apply such inks on lenses.

Currently, alternative to inks specifically designed for ophthalmic lenses uses are needed as these ink formulations need to be replaced due to the presence of compounds, in particular some solvents and catalysts, having Carcinogenic, Mutagenic or Toxic for Reproduction properties (i.e.; CMR substances) and to comply with the REACH EU regulation (Registration, Evaluation, Authorisation and Restriction of Chemicals). These CMR substances are used to improve the compatibility of the natural oils used in the ink to lenses' surface. Due to the new regulations these products will be discontinued.

Commercial CMR-free alternative products are not satisfactory as they lead to the occurrence of ghost image defects. Thus, none of the commercial inks currently available are suitable.

US6051629A1 describes an ink composition using vinyl or acrylic homo, or copolymers as principal components. This composition however is not easily erased with usual solvents such as alcohol and water.

The present disclosure aims to provide an ink composition for stamp-marking on ophthalmic lenses that overcomes at least one of the above-mentioned drawbacks.

### SUMMARY OF THE DISCLOSURE

Therefore, one object of the invention is an ink composition for stamp-marking on an ophthalmic lens. The composition comprises at least:
- a compound of formula (I) 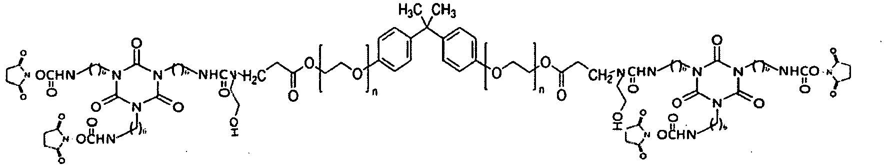 wherein n is 1 or 2,
- a drying aid, and
- at least one pigment.
The ink composition of the present disclosure is advantageously a "one component" system. That means that the ink composition is ready to be used by the end-user. Advantageously no cumbersome additional step, such as the mixing of components is to be carried out. Thus, it can be applied easily and conveniently. The compound of formula (I), or compound (I) is a polyurea-urethane copolymer. At each end of its chain, it comprises one urethane group which enables the formation of a hard film. The drying aid allows to adjust the drying time of the marking ink. A fast drying time, i.e. within 4 to 7 hours, is aimed at.

It should be noted that, the inventors tried first using an epoxy resin, instead of a compound of formula (I). However, the compatibility with the lens and the ink-applying stamp was not good and the composition was unstable. Therefore, according to one particular embodiment of the invention, the composition of the invention does not comprise an epoxy resin. Surprisingly, the compound of formula (I) provides a good compatibility with the lens and the stamp. Further, the ink composition according to the invention is more stable and is also versatile as it provides an ink with a good adhesion on most lens substrates such as coated & uncoated CR39 (also called Orma), polycarbonate, MR8 or MR7 made from polythiol-urethane resin are sold by Mitsui. Furthermore, the compound of formula 1 dissolves easily in solvents, in particular in standard solvents such as water and alcohols.

Moreover a compound of formula (I) complies with environment, health and safety (EHS) rules and is therefore environmentally friendly.

The relative amount of the compound having formula (I) over the total weight of the composition might range from 15.0 % to 40.0 %, preferably it ranges from 20.0 % to 35.0 % and more preferably between 21.0 and 25.0 % and preferably is about 22.5 %.

The pigment may be for example of the type Pigment Red 254 or Pigment yellow 151 , or similar types, such as Hostaperm Yellow H4G-EDS VP 3620, from Clariant Plastics and Coatings, CI Pigment Red 254, Cl.PIGMENT YELLOW 151, or a pigment mixture comprising one or more of these pigments, or other pigments for different base colors. The ink composition may comprise a mix of Hostaperm Yellow H4G-EDS VP 3620, CI Pigment Red 254 and Cl Pigment Red 254, or a mix of CI.PIGMENT YELLOW 151 and CI Pigment Red 254. The relative amount over the total weight of the composition of Hostaperm Yellow H4G-EDS VP 3620, CI Pigment Red 254, CI.PIGMENT YELLOW 151 might be approximately 10.5 wt. %, in the range of 0.015 to 0.14 wt % and approximately 13.7 wt. %.

The ink composition can advantageously comprise a surfactant such as BYK 3440 or BYK3455. The relative amount over the total weight of the composition of BYK 3440 or BYK3455 may be about 2.6 wt. % and about 1.95 %, respectively.

According to one preferred embodiment, n=1.

According to another preferred embodiment, the drying aid is a branched oxy-polyol, preferably a dendrimer, and preferably having the formula (II)

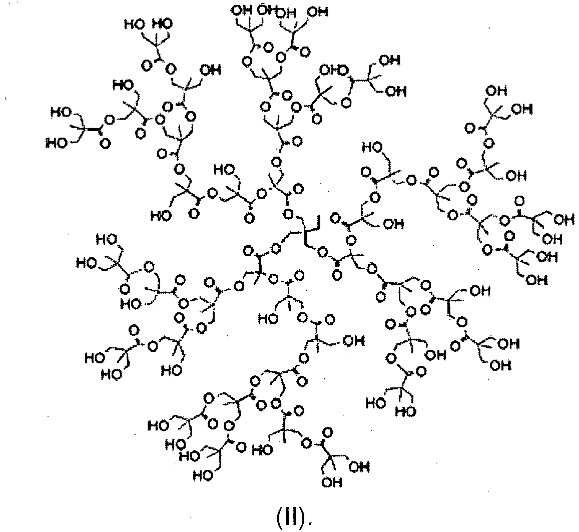

As a drying aid, the inventors tried to use Cellulose Acetate Butyrate or Jeffamine 2000 (polypropylene glycol diamine). However, the obtained composition was not stable, and was associated with cosmetic defects on lens. Hence, according to a particular embodiment of the invention, the composition does not comprise a cellulose acetate butyrate and/or a polypropylene glycol diamine. A branched oxy-polyol such as a compound having formula (II) provides a stable composition, and improves the aspect of the marked lenses, i.e. there is very little or no ghost image.

Furthermore, as for the compound of formula (I) a branched oxy-polyol, such as a compound having formula (II), is compliant with standard EHS rules.

The relative amount of the compound having formula (II) over the total weight of the composition may range from 2.0 wt. % to 4.0 %, preferably it ranges from 2,2 to 3,4 wt. %.

According to a further aspect of the invention, the composition may further comprise a solvent.

According to a further aspect of the invention, the composition advantageously comprises 1-methoxy-2-propanol (MP) and/or ethylene glycol diacetate (EGDA), preferably as solvents. A mixture of MP and EGDA is preferred. Such components provide for fast drying speeds and meets standards EHS rules.

The relative amount of ethylene glycol diacetate (EGDA) over the total weight of the composition may range from 7.0 wt. % to 21.0 wt.%. An amount of EGDA selected in this range is particularly effective to prevent the formation of ghost images upon its removal with solvents especially on lens material, such as uncoated MR8, uncoated MR7, uncoated 1.74 and/or uncoated PC. This lens material is advantageously an uncoated material. Preferably the amount of EGDA is at most 12.0 wt.%, more preferably at most 9.0 wt.%. A particularly effective concentration is around 8.6 wt. %. At these ranges, there is no ghost image after removal when assessed under R17 and ARC lamp, in particular when applied on ORMA UNC lens material.

For the same reasons, the relative amount of 1-methoxy-2-propanol (MP) over the total weight of the composition might range from 25.0 wt.% to 32.0 wt.%, preferably from 27.0 wt. to 30.4 wt.%. A particularly efficient concentration is about 27.7 wt.%.

The ink composition may also comprise ethylene glycol (EG) and/or propylene monomethyl ether acetate (MPA). Solvents such as EG and MPA, in particular in association with MP and EGDA, can provide for even faster drying speeds and meet standards EHS rules. The addition of EG and MPA in the ink composition allows to replace part of the EGDA and therefore decreases the relative amount of EGDA. Again, the formation of ghost images is prevented as well when the ink is applied on substrates such as uncoated ORMA lenses.

For the same reasons as exposed above, the relative amount of ethylene glycol diacetate (MPA) over the total weight of the composition may range from 15.0 wt. % to wt. 20.0 %, preferably it is approximately 18.0 wt.%.

For the same reasons, the relative amount of ethylene glycol (EG) over the total weight of the composition might range from 4.0 wt. % to wt. 6.0 %, preferably it is about wt. 5.2 %.

In total, the relative amount of total solvents over the total weight of the composition might range from 50,0 wt.% to 60,0 wt. %, preferably from 50.7 to 59.5 %. For the same reasons as exposed above, the relative amount of solvents over the total weight of the composition is preferably about 59.5%. For the same reasons, the solid content over the total weight of the composition might range from 37.0 wt.% to 48.0 wt.%, generally from 39.5 wt.% to 46.6 wt.%, preferably between 37.0 wt.% and 41.0 wt. %, and is more preferably about 39.5 wt.% or 40.5 %. The solid content was measured after drying the composition as described in the examples hereafter. The relative amount of solvents over the total weight of the composition can be chosen so that the ink composition has the targeted solid content, i.e., ranging from 37.0 wt.% to 41.0 wt. %, preferably is about 39.5 wt. %. Preferably, the viscosity of the ink composition is lower than 400 cps, preferably between 380 and 390 cps, most preferably about 395 cps.
Another object of the invention is a process to manufacture, prepare or obtain a compound of formula (I). The process comprises a step of mixing:
- a compound of formula (III), or Compound III: 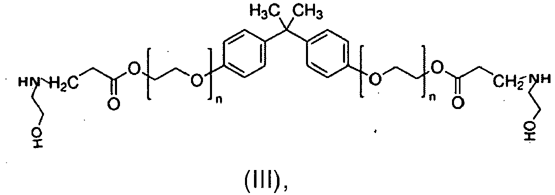 and
   - a compound of formula (IV), Compound IV: 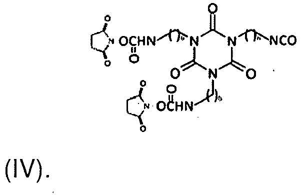
In formula (III), n is 1 or 2, preferably n=1.
The compound of formula (I), or Compound I, is prepared by reacting Compound III with Compound IV. Having Compound III provided with a small carboxy chain (i.e. n= 1 or 2, preferably n=1) increases the probability of obtaining a molecule with a urethane group at each end, i.e. of obtaining Compound I. Advantageously, Compound III and Compound IV are compatible with each other.

Advantageously, the molar ratio of compound III to Compound IV may range from 0.9 to 1.1, preferably is about 1:1.

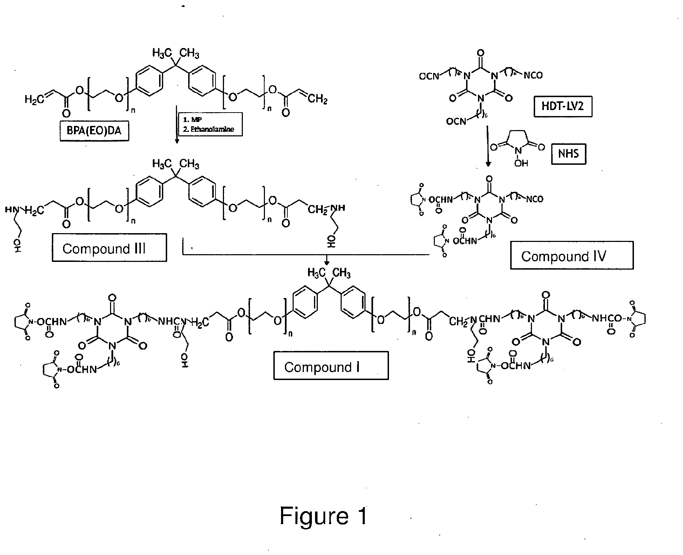

A schematic representation of a particularly preferred embodiment of the process of the invention is shown in figure 1. However, the process of the invention is not limited to this particularly preferred embodiment.

According to one embodiment and as also shown on figure 1, Compound III can be prepared by reacting bisphenol A ethoxylate diacrylate (BPA(EO)DA) and ethanolamine which are both widely available commercial materials. This reaction can take place in an organic solvent such as one comprising MP. MP is a particularly advantageous solvent as it has shown compatibility with BPA(EO)DA and ethanolamine as well as with Compound III and Compound IV. However other solvents or mixture thereof can be considered.

According to one embodiment, Compound III can be prepared by:
- contacting BPA(EO)DA and MP, preferably with stirring; (e.g., the mixture may be stirred for 10-20 minutes). The reaction can be carried out at room temperature, i.e. 18 to 25°C, and/or under atmospheric pressure. The stirring speed may be 300 to 500 rpm; and then
- adding ethanolamine into the mixture, preferably under continuous stirring for a suitable period of time, e.g. for two days under normal conditions of temperature and pressure.

According to a particular embodiment:
- the relative amount of BPA(EO)DA over the total weight of the composition can range from 38.0 wt.% to 58.0 wt.%, preferably from approximately 41.7 wt.% to 55.0 wt.%; the stoichiometric molar ratio of BPA(EO)DA to ethanolamine can range advantageously from 1:1.9 to 1:2.1, preferably is about 1:2;
- the relative amount of MP over the total weight of the composition can range from 28.0 WT.% to 50.0 wt. %, preferably from approximately 32.0 wt. % to 48.5 wt.%;
- the relative amount of ethanolamine over the total weight of the composition can range from 7.0 wt.% to 15.0 wt.%, preferably from approximately 9.9 wt. % to 13.0 wt. %.

According to one embodiment, as shown on figure 1, Compound IV is prepared by reacting the compound hexamethylene diisocyanate trimer (HDT-LV2) and N-Hydroxysuccinimide (NHS). These are widely commercially available compounds. This reaction can take place in a solvent which can comprise EGDA as a solvent, and eventually, with MPA as a cosolvent. EGDA and eventually MPA are particularly advantageous solvents as they have shown compatibility with HDT-LV2, NHS, Compound III and Compound IV.

According to one embodiment, Compound IV can be prepared by:
- contacting HDT-LV2 and EGDA, with eventually the addition of MPA, preferably with stirring (the mixture may be stirred for 10-20 minutes). The reaction can be carried out at room temperature, i.e. 18 to 25°C, and/or at atmospheric pressure. The stirring speed may be 300 to 500 rpm; and then,
- adding NHS into the mixture, preferably under continuous stirring, eventually with water cooling, for a suitable amount of time, e.g. preferably for two days, under the same conditions of time and pressure.

According to a particular embodiment:
- the relative amount of HDT LV2 over the total weight of the composition can range from 35.0 wt.% to 45.0 wt.%, preferably from approximately 37.6 wt. % to 42.0 wt.%; the stoichiometric molar ratio of HDT LV2 to NHS can be advantageously about 1:2;
- the relative amount of NHS over the total weight of the composition can range from 14,5 wt.% to 19,0 wt. %, preferably from approximately 15.8 wt.% % to 17,8 wt. %;

- the relative amount of EGDA over the total weight of the composition may range from 30.0 wt% to 48.0 wt.% , preferably from approximately 32.0 wt.% to 46.5 wt.%. In the presence of MPA, the relative amount of EGDA over the total weight of the composition may approximately be of 32.1 wt.%;
- the relative amount of MPA over the total weight of the composition may range from 0 wt.% to approximately 8.0 wt %.

According to one embodiment of the process of the invention:
- the compound of formula (III) is dissolved in a first liquid medium (e.g. 1-methoxy-2-propanol, MP) to obtain a first liquid;
- the compound of formula (IV) may be dissolved in a second liquid medium (e.g a medium comprising at least ethylene glycol diacetate (EGDA), preferably associated with propylene glycol monomethyl ether acetate (MPA) to obtain a second liquid); and

- said first liquid and said second liquid are then mixed together (mixing step).

According to one embodiment, this mixing step is carried out in the presence of a branched oxy-polyol, preferably having the formula (II)

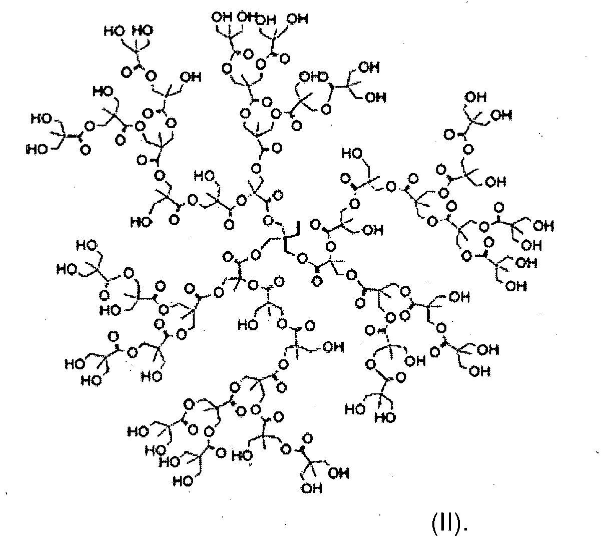

The presence of a branched oxy-polyol such as compound (II) has been shown to be very beneficial as it minimizes or prevents the formation of side products of the reaction. Thus, the efficiency of the reaction is substantially increased. It is possible that such a compound slows down the reaction.

The second liquid may thus advantageously comprise a branched oxy-polyol such as the compound of formula (II), prior to the mixing step of the second liquid with said first liquid.

The branched oxy-polyol, preferably having the formula (II), can be already in a solution, prior to be added into the second liquid. It can be dissolved in a solvent, or third liquid medium (e.g. 1-methoxy-2-propanol, MP) to obtain a third liquid.

Compound II can be dissolved into the third liquid medium, preferably using mechanical stirring. The mixture may be stirred for a suitable time period such as two days. The temperature of the reaction may range from room temperature, i.e. 18 to 25°C, to 50°C. The reaction may be carried out under atmospheric pressure. The stirring speed may be from 300 to 500 rpm.

According to one embodiment of the process of the invention:
- the relative amount of a branched oxy-polyol such as Compound II over the total weight of the third liquid can range from 19.0 wt.% to 22.5 wt.%, preferably from approximately 20.0 wt.% to 21.6 wt.%;
- the relative amount of MP over the total weight of the third liquid ranges from 77.0 wt.% to approximately 81.0 wt.%, preferably from approximately 78.4 wt. % to 80.0 wt.%.

According to one embodiment, prior to mixing the compound of formula (IV) with the branched oxy-polyol such as the compound of formula (II), the compound of formula (IV) is mixed with a solvent comprising 1-methoxy-2-propanol (MP) or a mixture of ethylene glycol (EG) and propylene glycol monomethyl ether acetate (MPA).

MP or EG and MPA can be added into the second liquid, preferably under stirring, for example at a stirring speed of 300 to 500 rpm and for a suitable period of time, e.g., for 5 to 10 minutes. The reaction can take place at room temperature and/or under atmospheric pressure.

Around 18.0 g of MP or 52.8 g of EG and 33.0 g of MPA can be added into the second liquid.

The branched oxy-polyol such as the compound of formula (II) can be added to the compound of formula (IV) by adding the third liquid into the second liquid drop by drop, and the obtained solution can be stirred between 700 and 900 rpm for a suitable amount of time such as 30 minutes. The reaction can take place at room temperature and/or under atmospheric pressure.

The solution of Compound II and Compound IV thus obtained may have the following amount over the total weight of the solution:
- about 56.7 wt.% of Compound IV,
- about 10.9 wt. % of EG,
- about 7.9 wt.% of MPA,
- about 1.0 wt.% of MP,
- about 23.5 wt. % of Compound II.

According to one embodiment, during the mixing step of the first liquid with the second liquid, the first liquid is added to the solution of Compound II and Compound IV, preferably drop by drop, preferably with water cooling. The mixture can be stirred between 700 and 900 rpm, for a suitable time such as two days. The reaction can be carried out at room temperature and/or under atmospheric pressure.

The invention also relates to a process for preparing an ink composition for stamp-marking on an ophthalmic lens and to the ink thus obtained. The process comprises the steps of:
- providing mixture of a compound of formula (I): 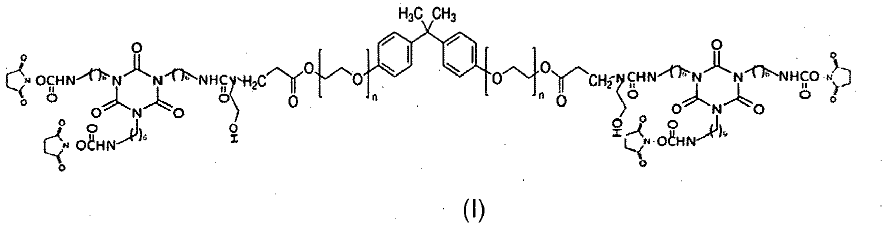 and
   a branched oxy-polyol, preferably having a formula (II). In formula (I), n= 1 or 2. Such compound I dissolves easily in solvents. Preferably, n=1. 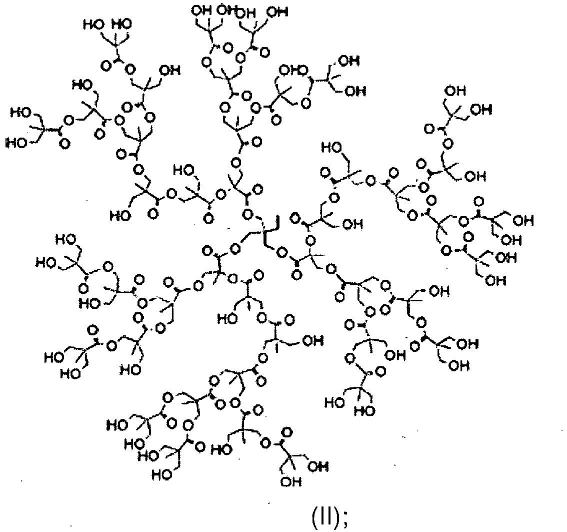 and
- adding at least one pigment (e.g. Cl Pigment Red 254, with Hostaperm Yellow H4G-EDS VP 3620 or Yellow pigment 151) to said mixture containing said compound of formula (I) and said compound of formula (II).

According to one embodiment, the mixture of a compound of formula (I) and a compound of formula (II) is obtained as described above.

Eventually, prior to adding at least one pigment, at least one solvent (e.g. MP and MPA) is added to said mixture containing said compound of formula (I) and said compound of formula (II). The pigment can be mixed, preferably under mechanical stirring (e.g., the stirring speed may be 300 to 500 rpm); the mixture may be stirred for a suitable period of time such as 1 to 2 hours. The temperature may be room temperature, i.e. 18 to 25°C, and the reaction can be carried out under atmospheric pressure.

At least one surfactant, for example a surfactant comprising or essentially consisting of 2-Methoxymethylethoxypropanol or a polyether modified siloxane, e.g. BYK 3440 or BYK 3455 by Byk Chemie GmbH, could be added to said mixture containing said compound of formula (I) and said compound of formula (II) with the at least one pigment. BYK 3455 is more environmentally friendly and has little or no CMR issues.

After the addition of the at least one pigment, eventually with the at least one surfactant, the mixture may be mechanically stirred (e.g., 300 to 500 rpm) for a suitable amount of time such as 30 minutes. The reaction can take place at room temperature and/or atmospheric pressure. Then the mixture can be advantageously milled, e.g. with glass beads for a suitable time, such as two to three hours. The milling steps can take place at room temperature and/or atmospheric pressure. As pigments are usually aggregated, such a milling step helps homogenisation, i.e., separating it into individual particles and dispersing it into the mixture. Thus, the marking ink solution is well dispersed.

Usually, after milling or grinding, the mixture is filtered to remove the glass beads.

The process for preparing an ink composition can comprise a further step of adding at least one solvent (e.g. a solvent comprising MP and MPA). The proportion of the solvent is advantageously so that the solid content of the composition is ranging from 37 wt.% to 41 wt.%.

The process for preparing an ink composition according to the invention can comprise a further step of stabilizing the composition by resting the composition for a suitable period which can range from one to two weeks before using and/or applying the composition.

The invention also encompasses the compounds, or their mixtures, which are used as precursors in the preparation of the compound of formula I as described therein.

In particular, an object of the invention is a compound of formula (III) as above described.

Another object of the invention is a compound of formula (IV), as above described.
A further object of the invention is the use of compound III and/or IV in the preparation of compound I as well as composition comprising at least one of said compound III and IV in association with a solvent, in particular a composition as above described.

Another preferred object of the invention is a compound of formula (I) As above described.

A further object of the invention if the use of Compounds I, II, III, and/or IV, and/or of a branched oxy-polyol (preferably a dendrimer), in the preparation of an ink composition as above described.

A further object of the invention is the use of the ink composition according to the invention and as above described in the marking of lenses and in particular ophthalmic lenses.

A further object of the invention is a method of providing temporary marking upon a lens, in particular an ophthalmic lens, said method comprising the step of applying an ink composition as described above upon a surface of the lens, in particular by jet spraying. Advantageously said method comprises a drying step, so that any solvent in the composition is removed, and eventually a removal step which may be carried out by applying a standard solvent such as alcohol to remove the marking.

A further object of the invention is a lens, preferably an ophthalmic lens, which comprises marking with the ink composition of the invention. This composition is advantageously virtually free of CMR solvents.

It should be noted that the branched oxy-polyol used in the invention is preferably a dendrimer. Its molecular mass can vary from .6500 to 8000 g.mole⁻¹.

### BRIEF DESCRIPTION OF THE DRAWINGS

Other advantages and features will become apparent upon reading the description of the following figures, among which:
- Figure 1 shows some process steps for preparing a compound of formula (I) according to a preferred embodiment of the invention.
- Figure 2 shows a diagram of various process steps for preparing an ink composition according to a first embodiment of the present invention,
- Figure 3 process for preparing an ink composition according to a second embodiment of the present disclosure,
- Figure 4 shows pictures of a lens according to the invention on which an ink composition according to the invention has been applied.

### DETAILED DESCRIPTION - EXAMPLES

**Table 1: List of compounds used for preparing exemplary ink formulations:**

| **Chemical name** | **CAS No.** |
|---|---|
| Bisphenol A ethoxylate diacrylate (BPA(EO)DA) | 64401-02-1 |
| 1-Methoxy-2-propanol (MP) | 107-98-2 |
| Ethanolamine | 141-43-5 |
| hexamethylene diisocyanate trimer (HDT-LV2) | 28182-81-2 |
| N-Hydroxysuccinimide (NHS) | 6066-82-6 |
| Ethylene glycol diacetate (EGDA) | 111-55-7 |
| CYD H40, compound of formula II, by Weihai CY Dendrimer Technology Co., Ltd | 326794-48-3 |
| BYK-3440 | 34590-94-8 |
| Hostaperm Yellow H4G-EDS VP 3620 | 31837-42-0 |
| CI Pigment Red 254 | 84632-65-5 |
| Propylene glycol monomethyl ether acetate (MPA) | 108-65-6 |
| Cl.PIGMENT YELLOW 151 | 31837-42-0 |
| Ethylene glycol (EG) | 107-21-1 |

Compound BYK-3455 is a polyether-modified polydimethylsiloxane available from BYK, Germany. It is a fluorine-free, silicone-containing additive for improving substrate wetting and leveling in aqueous systems and solvent-free UV coatings.

These compounds are meeting the standard requirements regarding health and safety and are environmentally friendly.

### Example 1: An ink composition according to a first embodiment

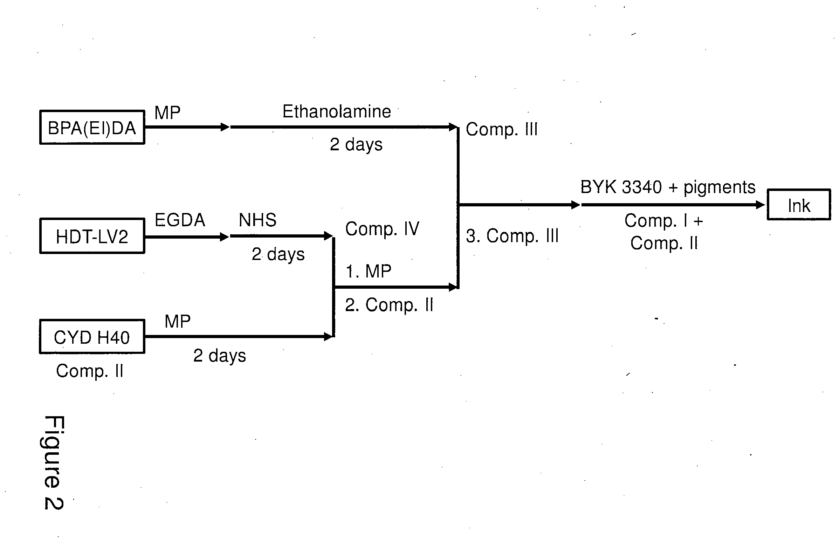

A marking ink according to a first embodiment of the present disclosure has been prepared following steps depicted in figure 2.

### Synthesis of Compound III

**Table 2: Compounds for the synthesis of Compound III**

| **Chemicals** | **Weight (g)** | **% Weight** |
|---|---|---|
| BPA(EO)DA | 15.5 | 55.02 |
| MP | 9.0 | 31.95 |
| Ethanolamine | 3.67 | 13.03 |
| Total | 28.17 | 100 |

In this embodiment, BPA(EO)DA with n=1 was used. BPA(EO)DA and MP, were added into a reactor stirred for 10-20 minutes at room temperature, i.e. 18 to 25°C, and atmospheric pressure, at a stirring speed of 300 to 500 rpm to reach complete dissolution of BPA(EO)DA. Ethanolamine was then added into the mixture and continuously stirred for 2 days in the same conditions. A solution of Compound III was obtained.

### Synthesis of Compound IV

**Table 3: Compounds for the synthesis of Compound IV**

| **Chemicals** | **Weight (g)** | **% Weight** |
|---|---|---|
| HDT LV2 | 32.76 | 37.63 |
| EGDA | 40.5 | 46.52 |
| NHS | 13.8 | 15.85 |
| Total | 87.06 | 100 |

HDT LV2 was dissolved in EGDA into a reactor by stirring the mixture for 10-20 minutes at room temperature and atmospheric pressure, at a stirring speed of 300 to 500 rpm. Then, NHS was added directly into the mixture and water cooling provided, in order to maintain the temperature below 35°C and thus avoid side reactions. The mix was continuously stirred for 2 days, in the same conditions. The solid NHS dissolved slowly into the solution and reacted with HDT LV2 to form a solution of Compound IV.

### Solution of Compound II

**Table 4: Materials for preparing a solution of Compound II**

| **Chemicals** | **Weight (g)** | **% Weight** |
|---|---|---|
| CYD H40 | 6.9 | 21.63 |
| MP | 25 | 78.37 |
| Total | 31.9 | 100 |

Compound II was dissolved in MP, with a stirring speed of 300 to 500 rpm at a temperature ranging from room temperature (18 to 25°C) to 50°C and atmospheric pressure, for two days, to get a solution of compound II.

### Preparing a solution of Compound II and Compound IV

18.0 g of MP was added into the solution of Compound IV previously obtained. It was stirred at a stirring speed of 300 to 500 rpm for 5 to10 minutes at room temperature and atmospheric pressure. After that, the solution of Compound II was added into the solution of compound IV drop by drop. 5 g of MP was then used to rinse the reactor of which has contained the solution of the of Compound II, to make sure that the entire amount of Compound II was transferred into compound IV solution. The obtained solution was stirred between 700 and 900 rpm for 30 minutes at room temperature and atmospheric pressure.

### Preparation of Compound I, preparation of a solution containing both Compound I and Compound II

The solution of Compound III previously obtained, was added into the solution comprising Compound IV and Compound II drop by drop. The reactor was cooled using water cooling, in order to maintain the temperature below 35°C and thus avoid side reactions 4 g of MP was used to rinse the reactor having contained the solution of Compound III and this added to the reactor containing Compound II, III and IV. This mixture was then stirred at a speed of between 700 and 900 rpm for 2 days at room temperature and atmospheric pressure. This resulted in a mixture comprising both Compound I and Compound II.

### Preparing an ink composition

**Table 5: Materials for the synthesis of an ink composition**

| **Ingredients** | **Weight (g)** | **% Weight** |
|---|---|---|
| Solution containing Compound I and Compound II | 174.14 | 86.93 |
| Hostapern Yellow H4G EDS VP 3620 | 21.0 | 10.48 |
| CI pigment red 254 | 0.03 | 0.015 |
| BYK 3440 | 5.16 | 2.575 |
| Total | 200.33 | 100 |

The pigments Hostaperm Yellow H4G-EDS VP 3620 and CI Pigment Red 254 were then added to the mixture comprising Compound I and Compound II as well as 5.16 g of the surfactant BYK 3440. 200 g of a glass bead having a diameter of 2mm were then added to the mixture which was was placed into a Vibro Shaker of S&S Industries, India, for 2 to 3 hours at room temperature and atmospheric pressure. As pigments are usually aggregated, this milling step with glass beads helps separating the aggregate into individual particles and provide an homogenous dispersion. After milling, the pigment particle size is about from 1 to 5 µm. After milling, the solution was filtered to remove the glass beads and a well dispersed marking ink solution provided.

The ingredients of marking ink composition are listed in Table 6.

**Table 6: Ingredients of marking ink composition:**

| **Ingredients** | **Weight (g)** | **% Weight** |
|---|---|---|
| BPA(EO)DA | 15.5 | 7.74 |
| Ethanolamine | 3.67 | 1.83 |
| HDT LV2 | 32.76 | 16.35 |
| EGDA | 40.5 | 20.22 |
| NHS | 13.8 | 6.90 |
| CYD H40 | 6.9 | 3.44 |
| MP | 61 | 30.45 |
| Hostaperm Yellow H4G EDS VP 3620 | 21.0 | 10.48 |
| CI pigment red 254 | 0.03 | 0.015 |
| BYK 3440 | 5.16 | 2.575 |
| Total | 200.33 | 100 |

### Example 2: An ink composition according to a second embodiment

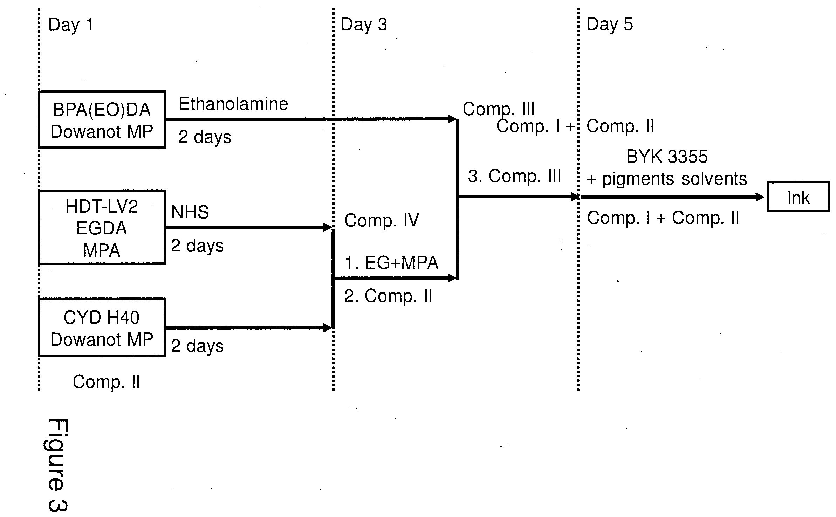

A marking ink according to a second embodiment of the invention has been prepared following steps shown in figure 3. It was prepared in the same way as for the ink composition of Example 1, except that the solvent system and the surfactant used were not the same.

### Synthesis of Compound III

**Table 7: Materials for the synthesis of Compound III**

| **Chemicals** | **Weight (g)** | **% Weight** |
|---|---|---|
| BPA(EO)DA | 54.52 | 41.66 |
| MP | 63.44 | 48.48 |
| Ethanolamine | 12.91 | 9.86 |
| Total | 130.87 | 100 |

Indeed, Compound III was prepared exactly the same way as for ink composition of Example 1, except for the weight percentage for each ingredient. The weight percentage for the MP solvent, was increased, as compared to that of the solution of Compound III of Example 1. As a consequence as shown hereafter, the results in terms of ghost images' occurrences were improved. In this embodiment, BPA(EO)DA with n=1 was also used.

### Synthesis of Compound IV

**Table 8: Compounds for the synthesis of Compound IV**

| **Chemicals** | **Weight (g)** | **% Weight** |
|---|---|---|
| HDT LV2 | 115.44 | 42.09 |
| EGDA | 88.0 | 32.08 |
| MPA | 22.0 | 8.02 |
| NHS | 48.84 | 17.81 |
| Total | 274.28 | 100 |

The solution of compound IV was prepared exactly the same way as for the one described in Example 1, except that the MPA is partially replaced by EGDA. MPA was introduced into the reactor at the same time as EGDA.

### Preparing a third liquid comprising Compound II

**Table 9: Compounds for the synthesis of a solution of compound II**

| **Chemicals** | **Weight (g)** | **% Weight** |
|---|---|---|
| CYD H40 | 22.72 | 20 |
| MP | 90.88 | 80 |
| Total | 113.6 | 100 |

This solution was prepared exactly the same way as the one described in in Example 1. Only the weight percentage for each ingredient was changed.

### Preparing a solution of Compound II and Compound IV

The solution was prepared exactly the same way as for the one described in Example 1. However EG and MPA were used instead of MP.In addition, the total weight percentage of the solvents was increased, as compared to the one present in the solution of Compound II and Compound IV of Example 1. However, the molar ratio of Compound IV to Compound II remains the same. More precisely, 52.8 g of EG and 33.0 g of MPA were added instead of 18.0 g of MP as shown in Table 10. As for rinsing the reactor, a mixture of 5 g. of MP and 5 g. of MPA was used, instead of 5 g. of MP as described in Example 1.

**Table 10: composition of a mixture of Compound II and Compound IV**

| **Chemicals** | **Weight (g)** | **% Weight** |
|---|---|---|
| Compound IV | 274.28 | 56.70 |
| EG | 52.8 | 10.92 |
| MPA | 38.0 | 7.86 |
| MP | 5.0 | 1.03 |
| Compound II | 113.6 | 23.49 |
| Total , | 483.68 | 100 |

### Preparation of Compound I, preparation of a solution containing both Compound I and Compound II

**Table 11: Compounds for the preparation of a solution containing both Compound I and Compound II**

| **Chemicals** | **Weight (g)** | **% Weight** |
|---|---|---|
| Solution of Compound II and Compound IV | 483.68 | 78.07 |
| Compound III | 130.87 | 21.12 |
| MP | 5.0 | 0.81 |
| Total | 619.55 | 100 |

This step was carried out in the same way as for Example 1 and 5 g of MP were used to rinse the Compound III's reactor to make sure that all the first liquid was transferred to the solution comprising Compound II and Compound IV. The total weight percentage for the solvents was increased, as compared with the one in the solution containing both Compound I and Compound II of Example 1. However the molar ratio of Compound I to Compound II is the same. The mixture was stirred in the same conditions as described in Example 1, to form a mixture containing both Compound I and Compound II. Therefore, in this embodiment, like in the one of example 1 and for the same reason, the mixing step was carried out in the presence of Compound II.

### Preparation of the ink composition

**Table 12: Compounds for the synthesis of the ink composition**

| **Ingredients** | **Weight (g)** | **% Weight** |
|---|---|---|
| Solution containing both Compound I and Compound II | 619.55 | 60.39 |
| MP | 120.00 | 11.70 |
| MPA | 125.00 | 12.18 |
| Yellow pigment 151 | 140 | 13.64 |
| CI pigment red 254 | 1.40 | 0.14 |
| BYK 3455 | 20 | 1.95 |
| Total | 1025.95 | 100 |

In this embodiment, prior to adding pigments, solvents (e.g. MP and MPA) were added to the mixture containing compound of formula (I) and compound of formula (II). MP and MPA were added into the mixture, and stirred for 1 to 2 hours at room temperature and atmospheric pressure and at a stirring speed of 300 to 500 rpm. The pigments Yellow pigment 151 and Cl Pigment Red 254, as well as surfactant BYK 3455 were then added to the mixture. The mixture was stirred for 30 minutes at room temperature and atmospheric pressure, and at a stirring speed of 300 to 500 rpm. Then 500 g of glass beads having a diameter of 2mm were added to the mixture which was placed into a Vibro Shaker and milled for 3 hours, in the same conditions described in Example 1. After milling, the glass beads were removed and a well dispersed liquid was obtained. A mixture 1:1 in weight of MP and MPA was added into the ink composition to achieve a solid content between 37 to 41 wt. %, with a viscosity lower than 400 cps. The solution was rested for 1 to 2 weeks after milling before it was used.

The various ingredients used to obtain the marking ink composition are listed in Table 13.

**Table 13: Ingredients of marking ink composition:**

| | **Chemical name** | **Weight (g)** | **Weight % SGMI-22** |
|---|---|---|---|
| Compound II and compounds for preparing Compound I | BPA(EO)DA | 54.52 | 5.31 |
| | 2-Aminoethanol | 12.91 | 1.26 |
| | HDT-LV2 | 115.44 | 11.25 |
| | NHS | 48.84 | 4.76 |
| | CYD H40 | 22.72 | 2.21 |
| Solvent system | MP | 284.33 | 27.71 |
| | EGDA | 88.00 | 8.58 |
| | MPA | 185.00 | 18.03 |
| | Ethylene glycol | 52.80 | 5.15 |
| Pigment system | Yellow pigment | 140.00 | 13.65 |
| | CI Red Pigment | 1.40 | 0.14 |
| Surfactant | BYK 3455 | 20.00 | 1.95 |
| Total (%) | | 1025.94 | 100 |
| Solid content (%) | | | 39.49 |
| Viscosity (cps) @200rpm | | | 395 |

As compared to the ink composition the first embodiment, more solvent has been necessary to obtain a composition ready to be applied. This change allows to obtain a lower viscosity and better results regarding the occurrence of ghost images, as shown hereafter.

### Example 3: Performance evaluation of ink compositions of Examples 1 and 2

The performances for ink compositions in Example 1 and 2 were evaluated.

The ability of a layer of ink to be dried and more particularly to be tack free (i.e. "tack-freeness"), was assessed by touch on the coated, dried ink and by observation that there are no stickiness or fingerprint on the ink surface.

ORMA is an acrylate resin with a refractive index of 1.498. It is also named Colombia resin 39 (CR 39). The adhesion on uncoated ORMA was measured using the crosshatch adhesion test carried out in accordance with standard ISTM 02-010. According to crosshatch test ISTM 02-010, a mark from 0 to 5 is given to the lens. With mark 0 or 1, the lens is acceptable (passes), whereas marks 2 to 5 are not acceptable (does not pass).

The ease to be removed by an MP solvent was assessed as follows. An ink marking was printed on multiple lenses which are then stored for 18 months to 2 years. Some lens samples are tested for ink removal at multiple intervals, such as after a week, a month, and regularly up to 18-24 months. The ink was removed by MP, IPA or acetate solvents. Ease to remove the ink with a solvent is then evaluated by sight.

Ghost image on uncoated ORMA was evaluated under R17 and ARC lamp after removal of the ink printed on the lens.

Contact angle on uncoated ORMA was measured by contact angle goniometer from Kruss-scientific.

Solid content was measured by Moisture analyser from Mettler Toledo, by adding 1 to 2 g. sample in the plate with temperature ramp time of 15 minutes and end temperatures of 150 or 160°C.

Viscosity was measured by viscometer from Broofield with various spindles depending on the viscosity.

**Table 14 shows the results of preliminary evaluations of the inks**

| Example | Example 1 | Example 2 |
|---|---|---|
| Solid content (%) | 46.60 | 39.49 |
| Viscosity (cps) | 413 | 395 |
| Contact angle on uncoated ORMA | 35 | 35 |
| Tack-free time (hours) | 4-5 | 4-5 |
| Adhesion on uncoated ORMA | good | good |
| Ease to be removed by MP | yes | yes |
| Ghost image on uncoated ORMA in a few weeks | yes | nil |

In both embodiments, tack freeness was achieved in 4 to 5 hours, showing a fast drying. A drying time between 4 to 7 hours is acceptable. Therefore, both compositions of Examples 1 and 2 gave good performances.

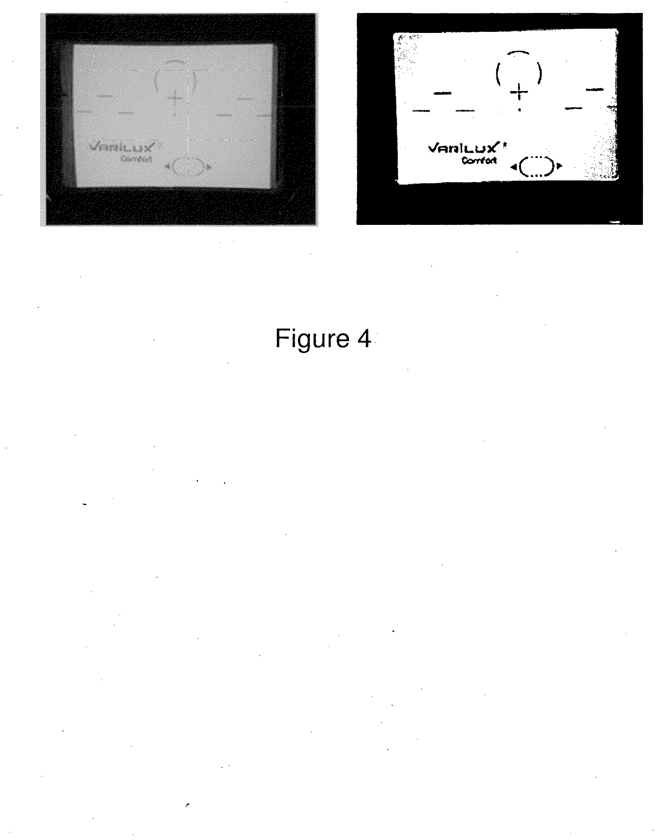

Figure 4 shows pictures of two lenses on which an ink according to the present invention has been applied. The left picture was taken after having printed the ink composition of Example 1 on an ORMA lens. The right picture was taken after having printed the same ink composition on a polythiol-urethane resin lens with a refractive index of 1.67.

### Example 4: Extended assessment of the ink composition of Example 1 and 2

A larger evaluation was conducted using the ink composition of Example 1. Two distinct batches (batch 1 and batch 2) of an ink obtained according to Example 1 were evaluated on different substrates (lenses).

The evaluations were performed on the following substrates:
The "UNC" in ORMA UNC, MR8 UNC, MR7 UNC, 1.74 UNC, 1.56 UV400 and PC UNC means those samples are all uncoated lenses.
MR8 UNC is a polythiol-urethane resin lens with a refractive index of 1.6.
MR7 UNC is a polythiol-urethane resin lens with a refractive index of 1.67.
1.74 UNC is a polythiol-urethane resin lens with a refractive index of 1.74.
1.56 UV400 is a poly allyl diglycol carbonate (CR39) resin lens with a refractive index of 1.56.
PC UNC is a polycarbonate resin lens with a refractive index of 1.59.

Transition S1 to Transition S8 are lenses coated with a multi-layer photochromic varnish as a top layer.

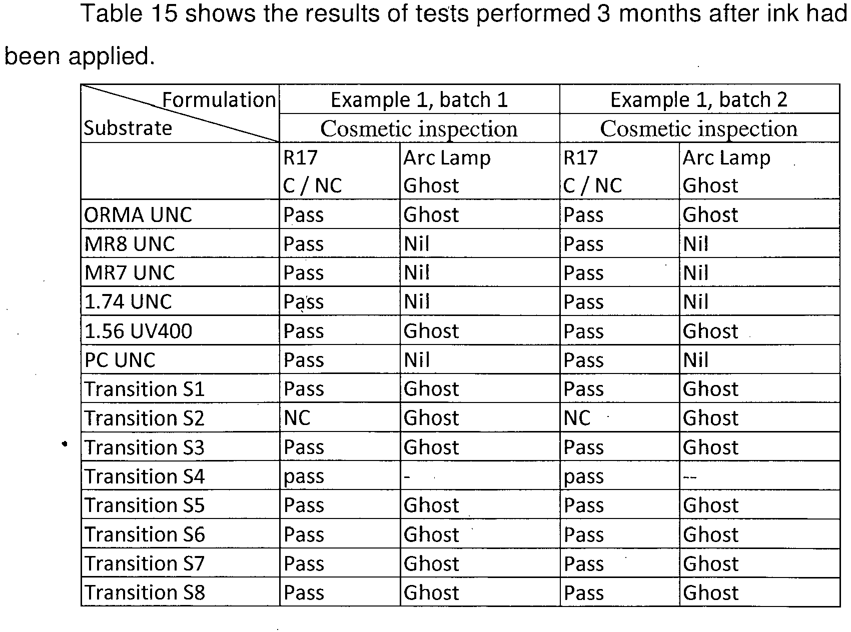

The ink composition of Example 1 shows good reproducibility and good performances on 12 different substrates, regarding ghost evaluation under R17. Likewise, satisfactory results when Arc lamp is used were obtained on MR8 UNC, MR7 UNC, 1.74 UNC and PC UNC. Therefore, the ink composition according to the first embodiment is particularly adapted to uncoated polythiol-urethane resin lenses, and for uncoated, polycarbonate resin, lenses.

With respect to the ink formulation of Example 2, it shows satisfactory results when Arc lamp is used on ORMA UNC and MR8 UNC. Therefore, this ink composition according to the second embodiment is particularly adapted to uncoated acrylate resin lenses (ORMA), and for uncoated, polycarbonate resin, lenses. Therefore, this preliminary evaluation suggests that replacing part of MP and EGDA in the solvent system with MPA and EG could provide ink compositions having good performances on most lenses.

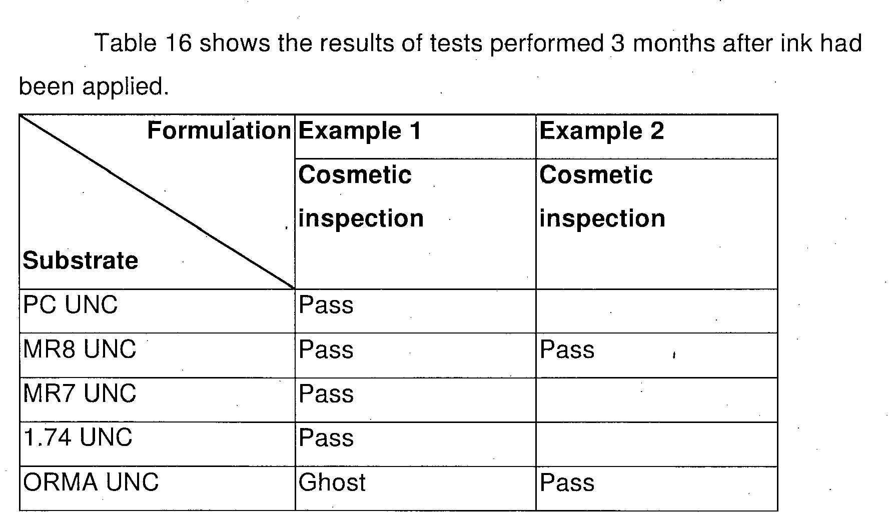

Although representative processes and inks have been described in detail herein, those skilled in the art will recognise that various substitutions and modifications may be made without departing from the scope of what is described and defined by the appended claims. For example, the ink composition could comprise other solvent and/or solvent mixes or different relative amount in order to be suitable for other type of lenses. The preparation of Compound I could be performed without the presence of Compound II, or Compound II could be added into the first liquid prior to mixing the first liquid and the second liquid. Even if in the disclosed embodiments, Compound I and Compound III have n=1, the disclosure is not limited to n=1, n could be 2.

## Claims

1. An ink composition for stamp-marking on an ophthalmic lens, said composition comprising at least:
- a compound of formula (I) 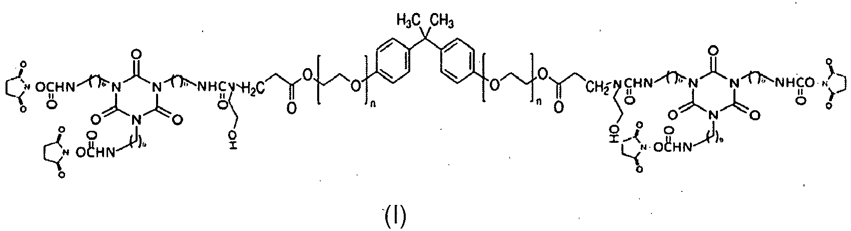 wherein n is 1 or 2,
- a drying aid, and
- at least one pigment.

2. The ink composition according to claim 1, the drying aid is a branched oxy-polyol, preferably having a formula (II)

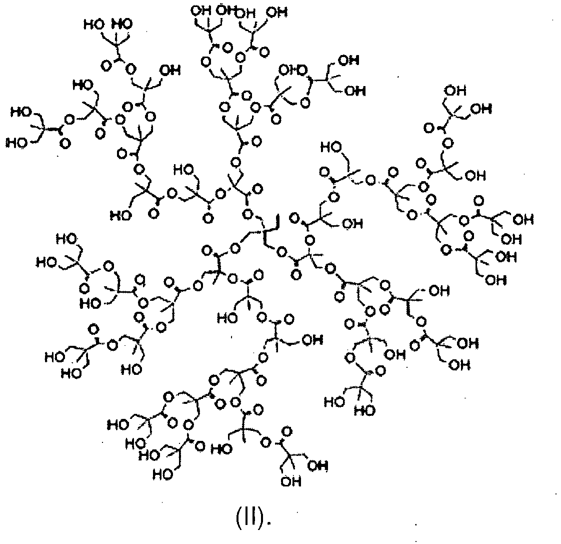

3. The ink composition according to any of claims 1 or 2, wherein said composition further comprises 1-methoxy-2-propanol and ethylene glycol diacetate, preferably as solvents.

4. The ink composition according to claim 3, wherein the relative amount of ethylene glycol diacetate over the total weight of the composition ranges from 7.0 % to 21.0 %, preferably it is at most 12.0 %, more preferably at most 9.0 %, the most preferably it is 8.6 %.

5. The ink composition according to any of claims 3 or 4, further comprising ethylene glycol and propylene monomethyl ether acetate.

6. The ink composition according to claim 5, wherein the relative amount of propylene monomethyl ether acetate over the total weight of the composition ranges from 15.0 to 20.0 %, preferably it is approximately 18.0 %.

7. A process for preparing a compound of formula (I) as defined in claim 1, said process comprising mixing
- a compound of formula (III): 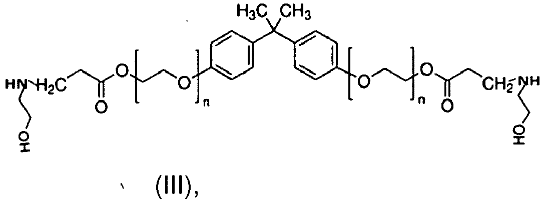 wherein n is 1 or 2; and
- a compound of formula (IV):

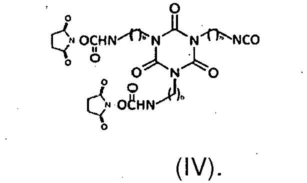

8. The process according to claim 7, wherein:
- the compound of formula (III) is dissolved in a first liquid medium (e.g. 1-methoxy-2-propanol) to obtain a first liquid;
- the compound of formula (IV) is dissolved in a second liquid medium (e.g at least ethylene glycol diacetate, preferably associated with propylene glycol monomethyl ether acetate) to obtain a second liquid; and
- said first liquid and said second liquid are mixed together during a mixing step.

9. The process according to any of claims 7 to 8, wherein said mixing step is carried out in the presence of a branched oxy-polyol, preferably having the formula (II)

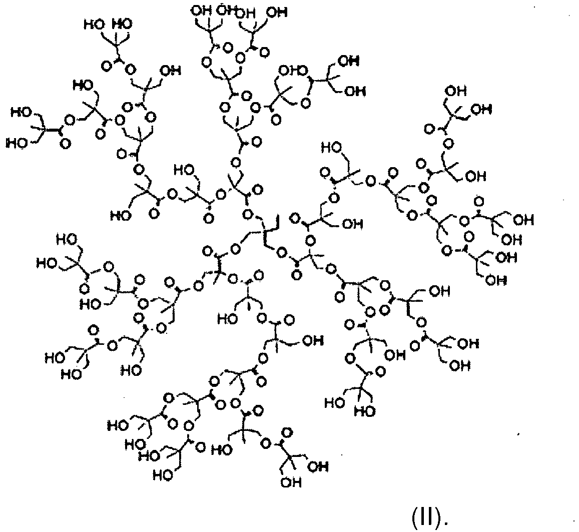

10. The process according to claim 9, wherein said second liquid further comprises the compound of formula (II) prior to the mixing step of second liquid with said first liquid.

11. A process for preparing a compound of formula (I) according to claim 9, in which, prior to mixing the compound of formula (IV) with the compound of formula (II), the compound of formula (IV) is mixed with 1-methoxy-2-propanol or a mixture of ethylene glycol and propylene glycol monomethyl ether acetate.

12. A process for preparing an ink composition for stamp-marking on an ophthalmic lens, said process comprising:
- providing mixture of a compound of formula (I): 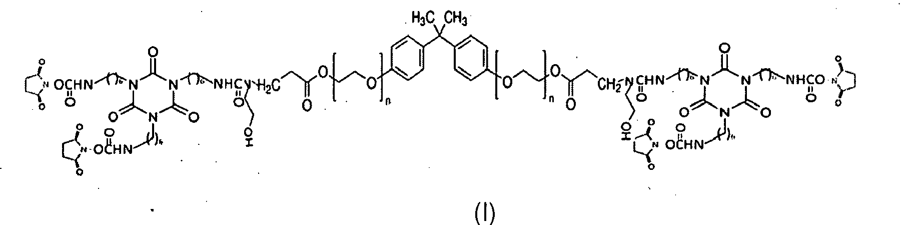 wherein n is 1 or 2; and
a branched oxy-polyol, preferably having a formula (II) 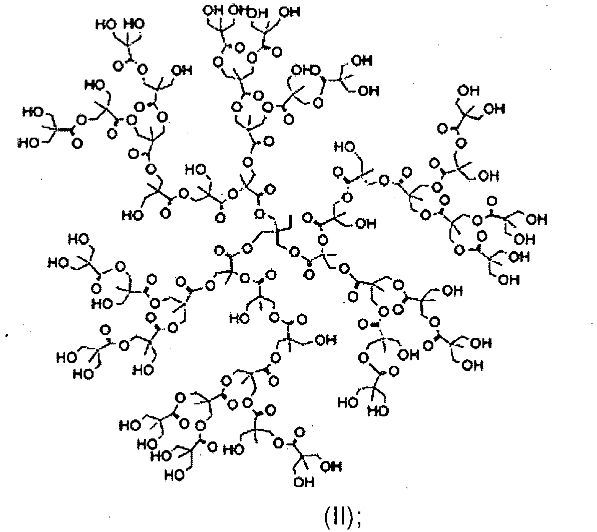 and
- adding at least one pigment to said mixture containing said compound of formula (I) and said compound of formula (II).

13. The process according to Claim 12, wherein said mixture of a compound of formula (I) and a compound of formula (II) is obtained according to the process of any one of Claims 8 to 10.

14. A compound of formula (III) 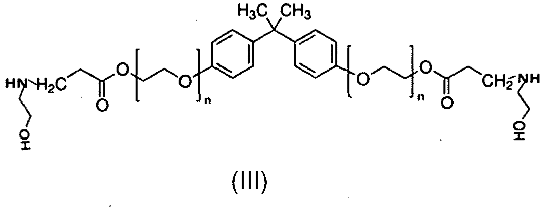 or a salt thereof,
wherein n is 1 or 2.

15. A compound of formula (IV) 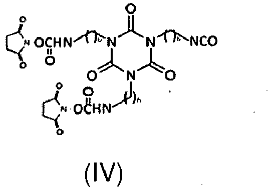 or a salt thereof.

16. A compound of formula (I) 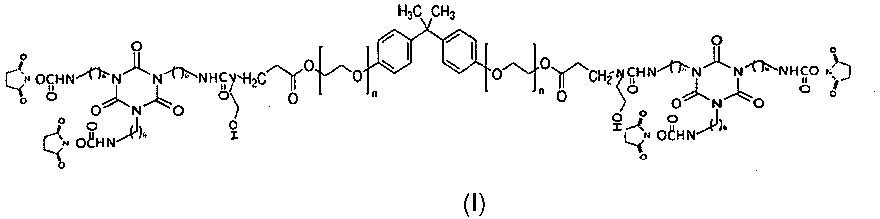 Wherein n is 1 or 2.

17. Use of
- a compound of formula (I) 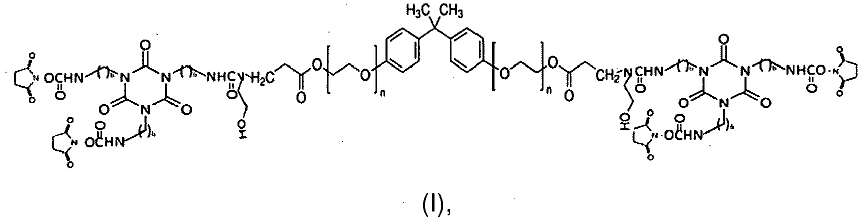
- a branched oxy-polyol, preferably having the formula (II): 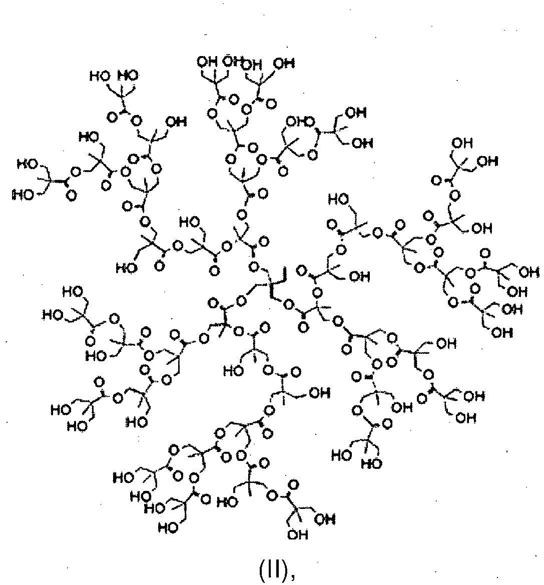
- a compound of formula (III) 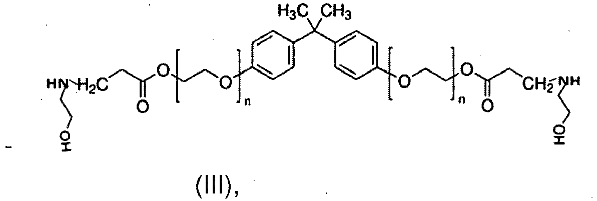 or a salt thereof, wherein n is 1 or 2, and/or
- a compound of formula (IV): 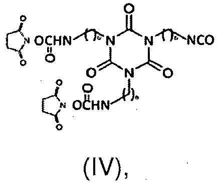
- or a salt thereof;
for the manufacture of an ink composition for stamp-marking on an ophthalmic lens.
